(19) Europäisches Patentamt / European Patent Office / Office européen des brevets

(11) **EP 2 848 282 B1**

(12) **EUROPÄISCHE PATENTSCHRIFT**

(45) Veröffentlichungstag und Bekanntmachung des Hinweises auf die Patenterteilung:
**10.08.2016 Patentblatt 2016/32**

(51) Int Cl.:
***A61N 1/08*** *(2006.01)*

(21) Anmeldenummer: **14180513.5**

(22) Anmeldetag: **11.08.2014**

(54) **Implantierbares Gerät**

Implantable device

Appareil implantable

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.09.2013 US 201361877298 P**

(43) Veröffentlichungstag der Anmeldung:
**18.03.2015 Patentblatt 2015/12**

(73) Patentinhaber: **BIOTRONIK SE & Co. KG**
**12359 Berlin (DE)**

(72) Erfinder:
- **Bartels, Klaus**
  **10115 Berlin (DE)**
- **Bitzer, Andreas**
  **8032 Zürich (CH)**
- **Büssing, Heinrich**
  **10317 Berlin (DE)**
- **Kolberg, Gernot**
  **12161 Berlin (DE)**
- **Jens, Rump**
  **12049 Berlin (DE)**
- **Knorr, Stefan**
  **10119 Berlin (DE)**

- **Täubert, Kerstin**
  **12589 Berlin (DE)**
- **Weiss, Ingo**
  **12435 Berlin (DE)**
- **Maxfiled, Michelle**
  **10967 Berlin (DE)**
- **Lenski, Hartmut**
  **15806 Zossen (DE)**
- **Trip, Erik**
  **7975 PG Uffelte (NL)**
- **Friedrich, Michael**
  **12049 Berlin (DE)**
- **Frenzel, Timo**
  **12435 Berlin (DE)**

(74) Vertreter: **Galander, Marcus et al**
**Biotronik SE & Co. KG**
**Corporate Intellectual Properties**
**Woermannkehre 1**
**12359 Berlin (DE)**

(56) Entgegenhaltungen:
| EP-A1- 2 486 952 | US-A1- 2008 033 497 |
| US-A1- 2008 243 218 | US-A1- 2010 211 129 |
| US-A1- 2012 078 333 | US-A1- 2012 109 261 |
| US-A1- 2013 204 335 | |

**Beschreibung**

Die Erfindung betrifft ein permanent oder temporär implantierbares medizinisches Gerät mit einem langgestreckten elektrischen Leiter.

Solche Geräte, beispielsweise Elektrodenleitungen für die Elektrostimulation, haben den Nachteil, dass sich ihr elektrischer Leiter in einem elektromagnetischen Wechselfeld zum Beispiel in einem Kernspintornografen erwärmen kann, weil solche elektromagnetischen Wechselfelder in dem elektrischen Leiter elektrische Ströme induzieren.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse, beispielsweise an das Gewebe (Myokard) des Herzens oder zum Abfühlen elektrischer Felder, um im Rahmen des sogenannten Sensings eine Aktivität, beispielsweise eine Herzaktivität abfühlen zu können.

Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen.

Die Elektrodenpole sind über einen oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen, an deren proximalem Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleiter bezeichnet.

Solche Funktionsleiter sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

US 2013/0204335 A1 offenbart eine für die Magnetresonanztomografie kompatible elektrische Schaltung für eine Leiteranordnung unter Verwendung von LC-Filtern.

In EP 2486952 A1 und US 20120078333 A1 werden ein medizinisches Leitersystem beschrieben, welches elektromagnetische Bandsperr-Filter nutzt.

US 20120109261 A1 offenbart ein implantierbares medizinisches Gerät mit einem Leiter, dessen Länge ungefähr ein Viertel der Wellenlänge eines Störsignals entspricht.

US 20100211129 A1 offenbart ein Gerät und eine Methode für den Schutz von implantierbaren medizinischen Geräten vor störenden Feldern.

US 20080243218 A1 offenbart für die Magnetresonanztomografie und für Radiofrequenzen kompatible Leiter.

US 20080033497 A1 beschreibt ein für die Magnetresonanztomografie kompatibles implantiertes medizinisches Gerät und Leiter.

Der Erfindung liegt die Aufgabe zugrunde, ein medizinisches Gerät und insbesondere eine Elektrodenleitung zu schaffen, welches möglichst wenig durch elektromagnetische Wechselfelder zu beeinträchtigen ist. Beispielsweise soll die Erwärmung einer Elektrodenleitung oder eines Katheters in einem Kernspintornografen reduziert werden.

Erfindungsgemäß wird diese Aufgabe durch ein temporär oder permanent implantierbares medizinisches Gerät mit mindestens einer langgestreckten elektrischen Leitung gelöst, die einen Funktionsleiter als erste elektrische Komponente oder als Teil einer ersten elektrischen Komponente enthält, welche mit wenigstens einem Elektrodenpol zur Abgabe von Therapiesignalen oder zur Erfassung von Diagnosesignalen verbunden ist; und die mindestens eine zweite elektrische Komponente enthält, die mit der ersten elektrischen Komponente elektrischen Kontakt hat und deren elektrische Länge mindestens einem Zehntel der Wellenlänge von elektromagnetischen Wellen im Radiofrequenzbereich entspricht.

An einem langgestreckten medizinischen Gerät wird somit die elektrische Funktionsleitung durch eine weitere elektrische Leitungskomponente derart ergänzt, dass am Ort der Auskopplung eines elektrischen Signals eine minimierte Amplitude einer eingekoppelten elektromagnetischen (EM) Welle anliegt.

Die Erfindung schließt den Gedanken ein, ein medizinisches Gerät, insbesondere eine Elektrodenleitung zu schaffen, die elektromagnetische oder magnetische Wechselfelder eingekoppelte Energie für mindestens eine bestimmte Wellenlänge so in der elektrischen Leitung transformiert, dass am Punkt der Auskopplung elektrischer Signale (z. B. Stimulation oder Wahrnehmung) die Amplitude eingekoppelter elektromagnetischer Wellen möglichst gering ist. Ein paralleler Einbau der wärmereduzierenden zweiten elektrischen Komponente ist dabei einer seriellen Anordnung vorzuziehen, da die Auswirkungen auf die Funktion des implantierbaren medizinischen Gerätes reduziert werden und eine Beschädigung der zweiten elektrischen Komponente kein Risiko für die Funktionalität des implantierbaren medizinischen Gerätes darstellt. Eine große Flexibilität in Bezug auf die geometrische Ausführung ermöglicht die Umsetzung in bereits vorhandenen und etablierten Designkonzepten.

Erfindungsgemäß wird somit ein implantierbarer Körper mit mindestens einer ersten, langgestreckten elektrisch leitfähigen Komponente als Funktionsleiter mit einem proximalen und einem distalen Ende vorgeschlagen, das an mindestens einer Stelle auf der distalen Seite elektrischen Kontakt zum Gewebe/ Blut hat, wobei die erste elektrische Komponente elektrischen Kontakt zu mindestens einer zweiten elektrischen Komponente hat, deren elektrische Länge mindestens einem Zehntel der Wellenlänge ($\lambda$/10) von elektromagnetischen Wellen im Radiofrequenzbereich entspricht.

Die erfindungsgemäße Lösung vermeidet Nachteile bekannter Lösungen, bei denen Filterelemente in den Elektrodenkörper integriert sind. Bei solchen Lösungen können bewährte Designkonzepte nicht mehr verwendet werden. Dadurch wird das Elektrodenleitungs-Design bzgl. seiner physiologischen Eigenschaften geschwächt. Die Elektrodenleitung wird größer und steifer. Das Risiko von Gewebereizung und Perforation steigt. Darüber hinaus befinden sich Filterelemente im therapeutischen Pfad. Dadurch wird die Langlebigkeit der Konstruktion gefährdet oder eingeschränkt. Typischerweise eingesetzte Bandstoppfilter sind frequenzselektiv.

Vorzugsweise ist das implantierbare medizinische Gerät eine Elektrodenleitung zum Anschluss oder als Teil eines Therapie- und/oder Monitoringgerätes. Ein Innenleiter der Elektrodenleitung kann dann beispielsweise als Funktionsleiter die erste elektrisch leitende Komponente darstellen. Das implantierbare medizinische Gerät kann aber auch ein passives Implantat, z.B. ein chirurgischer Nagel sein, der in diesem Fall auch einen - wenn auch atypischen - Funktionsleiter und somit die erste elektrische Komponente darstellt. Vorzugsweise besitzt die Elektrodenleitung ein an ein Therapie- und/oder Monitoringgerät anzuschließendes oder angeschlossenes proximales Ende und ein von diesem entferntes distales Ende, wobei der zumindest eine Elektrodenpol und die zweite elektrische Komponente am distalen Ende der Elektrodenleitung angeordnet sind.

Insbesondere ist es vorteilhaft, wenn die zweite elektrische Komponente um einen Längsabschnitt der Elektrodenleitung gewickelt ist.

Gemäß bevorzugter Ausführungsvarianten weist die zweite elektrische Komponente einen elektrischen Leiter auf, der mäanderartig gewunden oder helix- oder spiralartig gewickelt oder sowohl gewunden als auch gewickelt ist, so dass die zweite elektrische Komponente äußere Abmessungen aufweist, die wesentlich geringer sind, als die elektrische Länge des Leiters.

Vorteilhaft ist es auch, wenn die erste elektrische Komponente ein proximales und ein distales Ende aufweist, und die erste und die zweite elektrische Komponente an einem Kontaktpunkt miteinander elektrisch verbunden sind, der sich im Bereich des distalen Endes der ersten elektrischen Komponente befindet. Die zweite elektrische Komponente und insbesondere deren Leiter kann sich dann ausgehend vom Kontaktpunkt in proximale oder distale Richtung der ersten elektrischen Komponente erstrecken.

Im Folgenden werden weitere vorteilhafte Ausgestaltungen und Varianten genannt:

Vorzugsweise ist die zweite elektrische Komponente entlang ihrer Länge isoliert.

Ausgehend von einem Kontaktpunkt mit der ersten elektrisch leitenden Komponente kann die zweite elektrische Komponente in distaler Richtung oder in proximaler Richtung der Elektrodenleitung geführt sein. Es können auch zwei oder mehr zweite elektrische Komponenten vorgesehen sein, von denen dann beispielswese eine in proximaler und eine in distaler Richtung geführt ist. Beispielsweise kann die zweite elektrische Komponente parallel in zwei oder mehr Leiter aufgeteilt sein.

Die zweite elektrische Komponente kann auch nur abschnittsweise isoliert sein. So kann sie beispielsweise jenseits eines distalen Kontaktpunktes und/oder eines anderen möglichen Kontaktpunktes auf dessen proximaler Seite isoliert weiter geführt sein. Vorzugsweise hat die zweite elektrische Komponente eine elektrische Länge, die insbesondere einem Viertel der Wellenlänge ($\lambda$/4) elektromagnetischer Wellen im Frequenzbereich von 10MHz bis 400MHz entspricht.

Vorzugsweise liegt der Abstand zwischen der Stelle des elektrischen Kontaktes der ersten und zweiten elektrischen Komponenten (diese Stelle ist im Rahmen dieser Beschreibung auch als Kontaktpunkt bezeichnet) zu mindestens einem distalen elektrischen Ende der leitfähigen zweiten elektrischen Komponente in einer derartigen Größenordnung, dass sich die Abstände im Radiofrequenzbereich nicht makroskopisch vereinfachen lassen.

Vorteilhaft ist es beispielsweise, wenn der Abstand zwischen Gewebekontakt und mindestens einem Leitungsende der zweiten elektrischen Komponente $(2 * n + 1) * \lambda/4$ $(n \in \mathbb{N}_0)$ einer Wellenlänge $\lambda$ im Radiofrequenzbereich beträgt. Besonders bevorzugt liegt n im Bereich zwischen n=0 und n=2.

Das implantierbares medizinisches Gerät, also beispielsweise eine Elektrodenleitung, ist vorzugsweise so konfiguriert, dass im Einsatzfall ein Gewebekontakt im Abstand $n * \lambda/2$ einer Wellenlänge $\lambda$ im Radiofrequenzbereich

von mindestens einem Leitungsende der zweiten elektrischen Komponente stattfindet. n ist dabei eine natürliche Zahl $(n \in \mathbb{N})$, und zwar vorzugsweise im Bereich von n=1 bis n=2.

Die vorstehend beschriebenen Ausführungsvarianten können wenigstens zum Teil auch miteinander kombiniert werden. Dies gilt auch für die nachfolgend beschriebenen Ausführungsvarianten:

Die zweite elektrische Komponente kann beispielsweise parallel in zwei oder mehr Leiter aufgeteilt sein.

Das Ende des Leiters der zweiten elektrischen Komponente kann elektrisch offen sein. Alternativ kann das Ende des Leiters der zweiten elektrischen Komponente elektrisch kapazitiv oder induktiv oder galvanisch mit dem Gewebe verbunden sein. Hierzu kann das Ende des Leiters der zweiten elektrischen Komponente elektrisch mit dem Punkt des Gewebekontaktes, also zum Beispiel mit einem Elektrodenpol, verbunden sein. Folgende Ausführungsvarianten sind in diesem Zusammenhang bevorzugt:

- Der Leiter der zweiten elektrischen Komponente ist an mindestens einer Stelle mit dem Punkt des Gewebekontaktes (z.B. Elektrodenpol) durch ein oder mehrere kapazitiv wirkende Elemente verbunden.

- Der Leiter der zweiten elektrischen Komponente ist an mindestens einer Stelle mit dem Punkt des Gewebekontaktes (z.B. Elektrodenpol) durch ein oder mehrere induktiv wirkende Elemente verbunden.

- Der Leiter der zweiten elektrischen Komponente ist an mindestens einer Stelle mit dem Punkt des Gewebekontaktes (z.B. Elektrodenpol) durch eine Kombination von induktiv und kapazitiv wirkenden Elementen verbunden.

- Der Leiter der zweiten elektrischen Komponente ist an mindestens einer Stelle mit dem Punkt des Gewebekontaktes (z.B. Elektrodenpol) durch ein oder mehrere elektronische Schalter, Dioden, Transistoren, Thyristoren, Z-Dioden verbunden.

Vorzugsweise ist der Leiter der zweiten elektrischen Komponente ganz oder teilweise von einem dielektrisch wirksamen Medium umgeben, das vorzugsweise eine relative Permittivität ($\varepsilon_R$) größer 10 und besser noch größer 100 oder gar größer 1000 hat. Das dielektrisch wirksame Medium, mit dem die zweite elektrische Komponente Kontakt hat, besteht vorzugsweise ganz oder abschnittsweise aus einem der folgenden Werkstoffe oder einer Kombination davon: Glas, Bariumtitanat, Polyethylen, Siliziumoxid, Siliziumkarbid, Keramik, Piezowerkstoffe, Aluminiumoxid, Titanoxid, Tantaloxid, Halbleiterwerkstoffe, Kunststoffe mit Beimengungen der oben genannten Werkstoffe.

Vorzugsweise steht die Kombination aus zweiter elektrischer Komponente und dielektrischem Medium zusätzlich in Kontakt mit einer elektrisch leitfähigen Fläche. Diese kann dann als Groundplate wirken und erhöht die effektive Permittivität.

Der Leiter der zweiten elektrischen Komponente ist realisiert als eine der Ausführungen oder einer Kombination: Draht/Band, platinierter Draht/Band, gewendelter Draht/Band, Litze, Seil, gedruckter Leiter, geschnittener Leiter, geätzter Leiter, Draht-/Bandwolle, galvanisch abgeschiedener Leiter, geschlitzte leitfähige Fläche.

Leiterwerkstoffe können folgende Materialien oder Kombinationen oder Legierungen daraus sein: Kupfer, Silber, Gold, Stahl, MP35N, Kohlenstoff, leitfähige Kunststoffe, Aluminium, Niob, Molybdän, Tantal, Titan, Platin, Metalle.

Vorzugsweise ist das implantierbare medizinische Gerät für einen eingeschränkten Frequenz- und damit Wellenlängenbereich ausgelegt, und zwar vorzugsweise für einen Frequenzbereich eines elektromagnetischen Wechselfeldes von 40MHz bis 150MHz. Die Erfindung soll nun anhand von Ausführungsbeispielen mit Bezug auf die Figuren näher erläutert werden. Von diesen zeigt:

Fig.1: einen implantierbaren Herzstimulator und eine daran angeschlossene implantierbare Elektrodenleitung;

Fig. 2: eine aktiv fixierbare Elektrodenleitung;

Fig. 3: einen distalen Abschnitt einer passiv fixierbare Elektrodenleitung;

Fig. 4: ein Detail des distalen Endes der Elektrodenleitung aus Figur 3 mit um einen Elektrodenkopf gewickeltem elektrischen Leiter als zweiter elektrischer Komponente;

Fig. 5: ein Detail des distalen Endes einer aktiv fixierbaren Elektrodenleitung ähnlich der aus Figur 2 mit um einen zentralen Funktionsleiter gewickeltem elektrischen Leiter als zweiter elektrischer Komponente;

Fig. 6 und 7: ein Detail des distalen Endes einer aktiv fixierbaren Elektrodenleitung ähnlich der aus Figur 2 mit um ein Kopfgehäuse gewickeltem elektrischen Leiter auf einer flexiblen Leiterbahn als zweiter elektrischer Komponente;

Fig. 8 und 9: verschiedene Varianten von auf eine Folie aufgebrachtem elektrischen Leiter, der zusammen mit der Folie als flexibler Leiterplatte eine wickelbare zweite elektrische Komponente bildet; und

Fig. 10 a) bis d): verschiedene Varianten von einen helixartig gewickelten elektrischen Leiter aufweisenden zweiten elektrischen Komponenten.

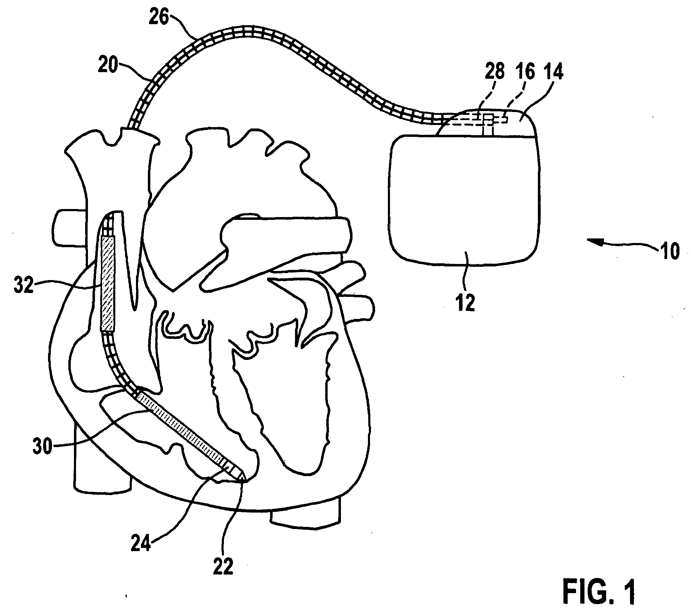

Fig. 1 zeigt als Beispiele implantierbarer medizinischer Geräte einen implantierbaren Herzstimulator 10 und eine daran angeschlossene implantierbare Elektrodenleitung 20.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können. Anstelle eines Herzstimulators kann auch ein reines Monitoringgerät zur Herzüberwachung vorgesehen sein. Typische Herzstimulatoren haben neben der Therapie- auch eine Monitoringfunktion und sind somit sowohl Therapie- als auch Monitoringgerät.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tip-Elektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tip-Elektrode 22 und die Ringelektrode 24, im Anwendungsfall die Elektrodenleitung 20, im Apex eines rechten Ventrikels eines Herzens befinden.

Sowohl die Tip-Elektrode 22 als auch die Ringelektrode 24 sind über jeweils wenigstens einen elektrischen Funktionsleiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren.

Wie weiter unten näher erläutert ist, können die elektrischen Leiter 26 in der Elektrodenleitung 20 in unterschiedlichen Längsabschnitten als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden, werden im Rahmen dieses Textes auch als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Funktionsleiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Wie den nachfolgenden Figuren zu entnehmen ist, kann insbesondere die Tip-Elektrode 22 auch als aktiv fixierbarer Elektrodenpol in Form einer korkenzieherartigen Helix ausgebildet sein. Die Elektrodenleitung 20 ist dann eine aktiv fixierbare Elektrodenleitung.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist. Weiterhin können solche Elektrodenleitungen unter fachüblicher Anpassung an die speziellen Erfordernisse des jeweiligen Anwendungsgebiets auch zur Stimulation und Ableitung von Signalen an Nerven, Gehirn, und anderen Organen oder für die Zuleitung von implantierbaren Sensoren Verwendung finden.

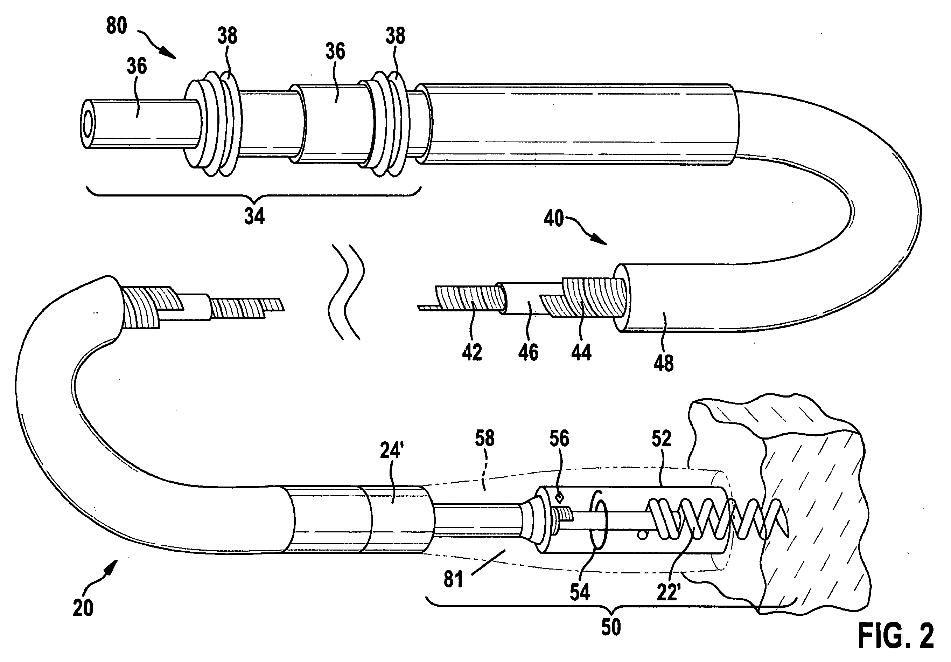

Fig. 2 zeigt eine aktiv fixierbare Elektrodenleitung 20 mit einem proximalen Ende 80 und einem distalen Ende 81. Diese Figur dient der Übersicht. Die dargestellte Elektrodenleitung 20 ist in der Mitte geschnitten, um nicht die volle Länge ausmalen zu müssen. Außerdem ist so die Elektrodenleitungskonstruktion gut sichtbar.

Die Elektrodenleitung 20 hat an ihrem proximalen Ende 80 eine Steckerseite, an der die Elektrodenleitung mit dem Gerät 10 verbunden werden kann und an ihrem distalen Ende 81 eine Kopfseite, bei der ein Elektrodenkopf 50 den Kontakt zum Gewebe herstellt. In dieser Ausführung ist die Elektrodenleitung bipolar aufgebaut und hat eine koaxiale Konstruktion.

Am proximalen Ende 80 ist ein zweipoliger Elektrodenleitungsstecker 34 mit zwei koaxialen Steckkontakten 36 und zwei Gummilippendichtungen 38 ausgebildet.

Zwischen dem proximalen Ende 80 und dem distalen Ende 81 erstreckt sich ein koaxialer, zweiadriger Leitungsabschnitt 40 mit einem helixartig gewundenen Innenleiter 42, einer zwischen diesem und einem helixartig gewickelten Außenleiter 44 angeordneten Isolation 46 sowie einer isolierenden Außenhülle 48. Der Innenleiter 42 und der Außenleitere 44 verbinden jeweils einen der Steckkontakte 36 mit jeweils einem Elektrodenpol am distalen Ende 81 der Elektrodenleitung 20'. Der zentrale Leitungsabschnitt 40 stellt somit eine Zuleitung zwischen dem Elektrodenleitungsstecker 34 und dem Elektrodenkopf 50 dar.

Am distalen Ende 81 der Elektrodenleitung 20' sind zwei Elektrodenpole angeordnet, nämlich ein erster Elektrodenpol in Form einer Ringelektrode 24' und ein zweiter Elektrodenpol in Form einer Tip-Elektrode 22', die als korkenzieherartige Helix ausgebildet ist und es erlaubt, die Elektrodenleitung 20' durch Einschrauben der Tip-Elektrode 22' aktiv in Herzgewebe einzuschrauben und somit das distale Ende 81 der Elektrodenleitung 20' aktiv zu fixieren. Im dargestellten Ausführungsbeispiel kontaktiert der Außenleiter 44' die Ringelektrode 24' und der Innenleiter 42' kontaktiert die korkenzieherartige, aktiv fixierbare Tip-Elektrode 22'. Distal der Ringelektrode 24' ist ein metallisches Kopfgehäuse 52 angeordnet, in dem die helixförmige Tip-Elektrode 22' geführt ist. Das Kopfgehäuse 52 ist auf seiner Außenseite elektrisch isoliert. Über eine Kontaktfeder 54 ist das Kopfgehäuse 52 mit dem Innenleiter 42 elektrisch verbunden.

In Fig. 2 ist außerdem ein Kontaktpunkt 56 dargestellt, an dem eine zweite elektrische Komponente (in Fig. 1 nicht dargestellt) elektrisch angeschlossen ist. Der Kontaktpunkt 56 ist in diesem Ausführungsbeispiel an dem Kopfgehäuse 52 angeordnet. Der Innenleiter 42' stellt in diesem Ausführungsbeispiel die erste elektrische Komponente dar. Der Kontaktpunkt 56 für die elektrische Kontaktierung der zweiten elektrischen Komponente am Elektrodenkopf liegt auf dem metallischen Kopfgehäuse 52 und liegt damit elektrisch gesehen etwa in Höhe des Gewebekontaktes.

Figur 2 zeigt somit ein aktiv fixierbares distales Elektrodenleitungsende 81 mit einem metallischen Kopfgehäuse 52, dass elektrisch mit der korkenzieherartig ausgebildeten und daher aktiv fixierbaren Tipp-Elektrode 22' verbunden ist. Das Kopfgehäuse 52 ist mit einem Dielektrikum beschichtet, dessen $\varepsilon_R$ in der Größenordnung von 10000 liegt. Dieses Dielektrikum verlangsamt eine im Kopfgehäuse fließende Welle der Frequenz von beispielsweise 64MHz derart, dass die Schwingungsdauer auf dem Weg vom Kontaktpunkt 56 zum Rand des Gehäuses einer Phase von $\pi/4$ entspricht. Als material für ein derartiges Dielektrikum eignen sich Ferroelektrika wie beispielsweise Bariumtitanat.

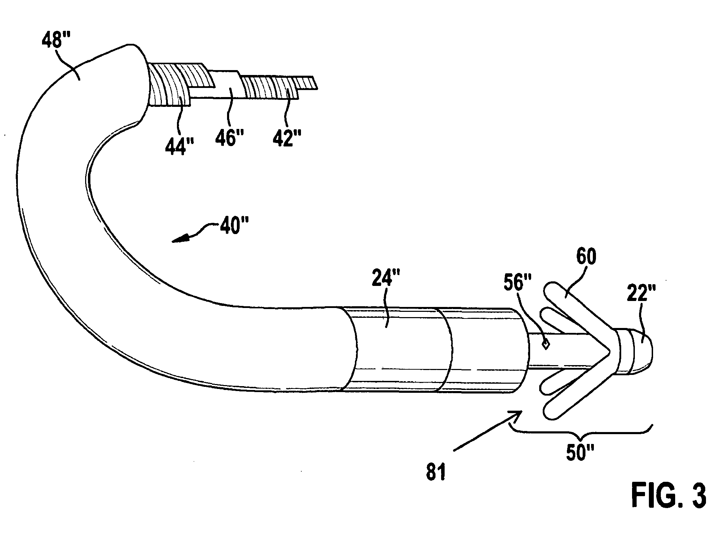

Fig. 3 zeigt einen distalen Längsabschnitt einer alternativen Elektrodenleitung 22". Auch diese weist einen Leitungsabschnitt 40" mit einem Innenleiter 42", einer zwischen diesem und einem Außenleiter 44" angeordneten Isolierung 46" sowie einer äußeren isolierenden Hülle 48" auf. Ebenfalls ist ein proximaler Elektrodenpol in Form einer Ringelektrode 24" vorgesehen. Eine Tip-Elektrode 22" ist als halbkugelförmige leitende Oberfläche an der distalen Spitze der Elektrodenleitung 22" vorgesehen. Etwas proximal der Tip-Elektrode 22" sind vier Tines 60 angeordnet. Diese Tines 60 bestehen beispielsweise aus weichem Kunststoff und dienen einer passiven Fixierung des distalen Endes 81" der Elektrodenleitung 40". Auch Fig. 3 zeigt einen Kontaktpunkt 56" zum Anschluss einer zweiten elektrischen Komponente.

Figur 3 stellt somit den distalen Bereich einer Elektrodenleitung 20 dar, die zur "passiven Fixierung" im Gewebe weiche Anker (Tines) 60 hat. Der Bereich hinter den Ankern 60 ist aus Metall und liegt auf gleichem Potenzial wie der Innenleiter (der auch hier die erste elektrische Komponente bildet). Auch hier ist ein sinnvoller Kontaktpunkt 56" für die Zweite elektrische Komponente eingezeichnet.

Die zweite elektrische Komponente kann einen Leiter, beispielsweise einen Draht, enthalten. Dieser kann um den jeweiligen Elektrodenkopf 50 oder 50" gewickelt sein. Vorzugsweise wird der Draht, der an einem Ende mit dem Elektrodenkopf 50 elektrisch verbunden ist, auf dem Elektrodenkopf 50 in eine Kopfisolation 58 eingebettet. Die Kopfisolation 58 besteht aus einem Material mit hoher Dielektrizitätskonstante (>>10), beispielsweise ein Ferroelektrikum, vorzugsweise auf Basis Bariumtitanat. Dadurch wird die Wellengeschwindigkeit im Draht 62", 44" oder 46" gesenkt und die Wellenlänge verkürzt. So ist es möglich, trotz verringerter Drahtlänge (< 100 cm) in einem Bereich zu bleiben, der sich nicht makroskopisch vereinfachen lässt.

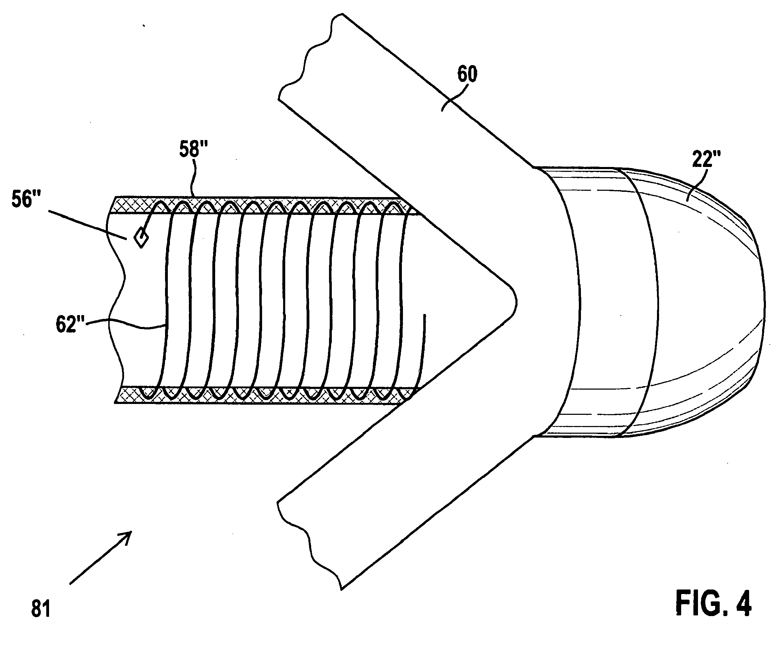

Fig. 4 zeigt ein Detail des distalen Endes 81" der Elektrodenleitung 40" aus Fig. 3 mit einer am Kontaktpunkt 56" angeschlossenen zweiten elektrischen Komponente in Form eines helixartig gewundenen, isolierten Drahtes 62". Fig. 4 stellt somit einen passiv fixierbaren Elektrodenkopf dar, der eine zweite elektrische Komponente enthält, die mit der ersten elektrischen Komponente (dem innenleiter 42") verbunden ist. Die zweite elektrische Komponente ist in diesem Fall ein isolierter metallischer Draht 62", der an dem Kontaktpunkt 56" mit dem Schaft des Elektrodenkopfes verbunden ist. Der Draht 62" ist außerhalb des Kontaktpunktes 56" gegenüber dem Schaft isoliert.

Durch die helixartig gewundene Form des isolierten Drahtes 62" als elektrischen Leiter der zweiten elektrischen Komponente sowie durch die Dielektrizitätskonstante $\varepsilon_R$ der den Draht umgeben Kopfisolation ist es möglich, dass die zweite elektrische Komponente geringe äußere Abmessungen hat und dennoch eine elektrische Länge, die mindestens einem Zehntel der Wellenlänge von elektromagnetischen Wellen im Radiofrequenzbereich entspricht. Die Wellenlänge elektromagnetischer Wechselfelder im Radiofrequenzbereich zwischen 10 MHz und 400 MHz beträgt im Vakuum zwischen 30 m (bei 10 MHz) und 75 cm (bei 400 MHz). Aufgrund der durch die Helixform bedingten Induktivität des elektrischen Leiters 64" und eines durch die Dielektrizitätskonstante des den elektrischen Leiter 64" umgebenden Isoliermaterials bedingten Kapazitätsbelag kann der elektrische Leiter 64" der zweiten elektrischen Komponente hinsichtlich seiner geometrischen Länge kürzer sein als es dem entsprechenden Bruchteil der Wellenlänge im Vakuum entspricht, weil die Wellenlänge bei gegebener Frequenz auf einem elektrischen Leiter der beschriebenen Art kürzer ist als im Vakuum.

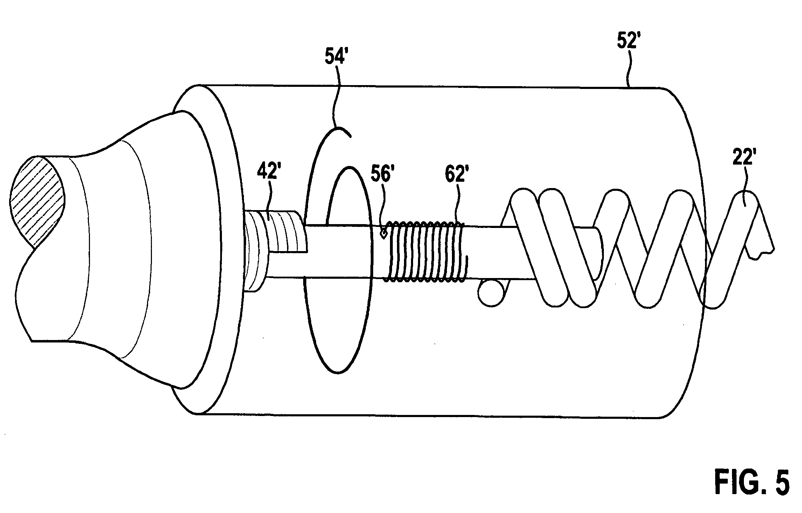

Fig. 5 Stellt einen aktiv fixierbaren Elektrodenkopf mit einem Schraubkopf dar, der eine zweite elektrische Komponente enthält, die mit dem Innenleiter 42' als erster elektrischer Komponente elektrisch verbunden ist. Zweite elektrische Komponente ist in diesem Fall ein isolierter metallischer Draht 62', der an dem Kontaktpunkt 56' mit der Achse des Elektrodenkopfes 50 verbunden ist. In einer anderen hier nicht dargestellten Ausführungsvariante ist der isolierte Draht außen auf das Kopfgehäuse gewickelt.

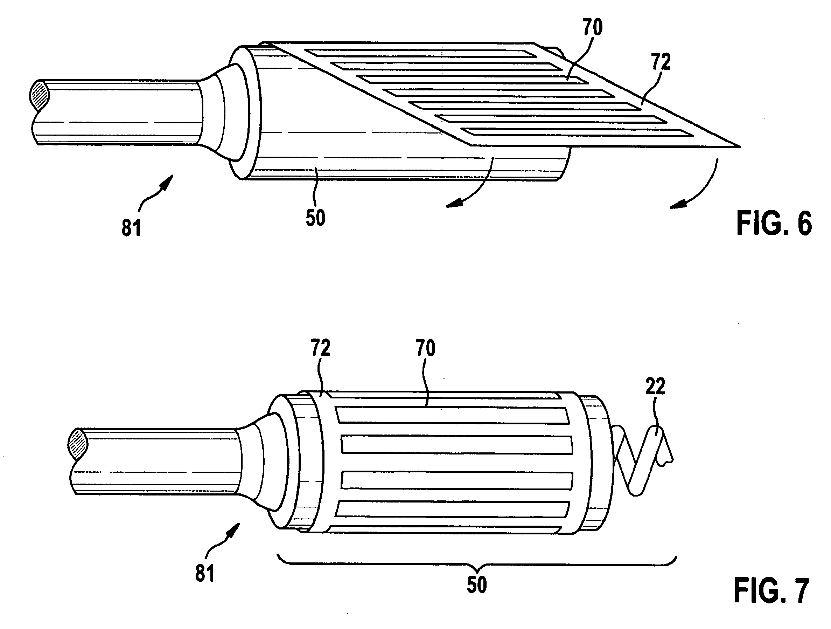

Figuren 6 und 7 zeigen das distale Ende 81 einer aktiv fixierbaren Elektrodenleitung 20 der in Figur 2 dargestellten Art. Bei dem in Figuren 6 und 7 dargestellten Ausführungsbespiel ist die zweite elektrische Komponente um den metallischen Elektrodenkopf 50 und konkret um das metallische Kopfgehäuse 52 gewickelt. Die zweite elektrische Komponente ist hierbei von einer gewundenen, leitfähigen Leiterbahn 70 auf einem flexiblen Trägermaterial 72, beispielsweise einer biegeweichen Folie, gebildet. Die Leiterbahn 70 ist am Kontaktpunkt 56 (siehe Figur 2) elektrisch angeschlossen und über diesen mit dem Innenleiter 42 und Elektrodenleitung 20 elektrisch verbunden. Figur 6 zeigt, wie das flexible Trägermaterial 72 mit der Leiterbahn 70 um den Elektrodenkopf 50 gewickelt werden kann und im Ergebnis die in Figur 7 abgebildete Struktur ergibt. Nicht dargestellt ist, dass die um den Elektrodenkopf 50 gewickelte zweite elektrische Komponente in ein Dielektrikum mit einer hohen relativen Permittivität $\varepsilon_R$ eingebettet sein kann.

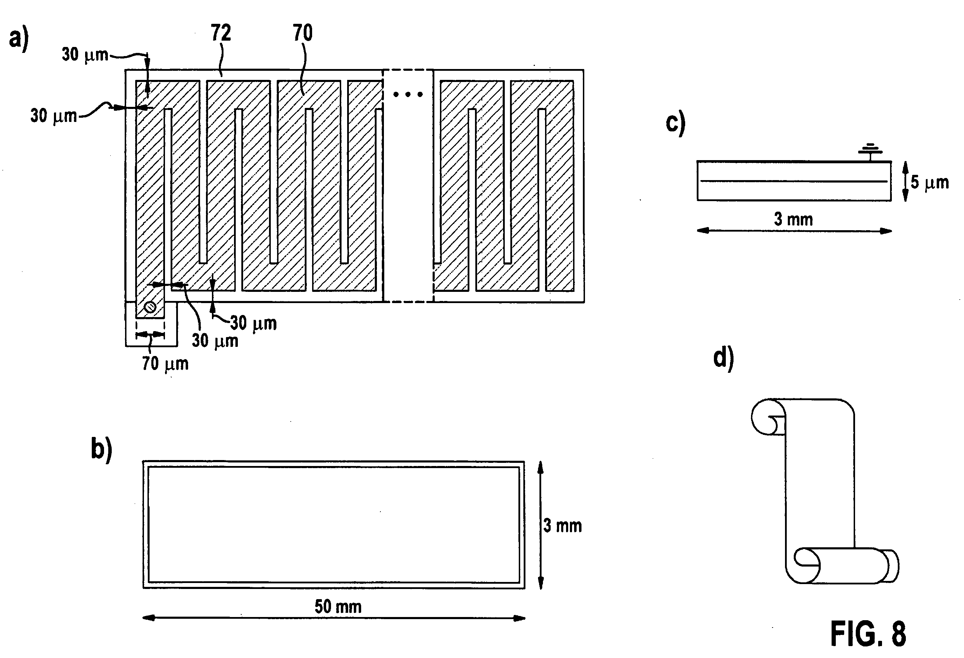

Figur 8 zeigt Details des flexiblen Trägermaterials 72 und der darauf befindlichen Leiterbahn 70, nämlich insbesondere potentielle Abmessungen dieser Komponenten. Dementsprechend kann das flexible Trägermaterial mit darauf angeordneter Leiterbahn zwischen 3 und 8 $\mu$m dick sein und eine Länge (in Längsrichtung der Elektrodenleitung betrachtet) zwischen 2 und 5 mm haben. Die abgewickelte Breite - im Beispiel 50 mm - hängt davon ab, wie groß die elektrische Länge der Leiterbahn 70 sein soll. Die Leiterbahn 70 hat in dem in Figur 8 dargestellten Ausführungsbeispiel eine Breite von 70 $\mu$m. Geeignet sind Breiten zwischen 50 und 100 $\mu$m. Wie Figur 8 ebenfalls zu entnehmen ist, verläuft die Leiterbahn 70 auf dem flexiblen Träger 72 zickzackförmig.

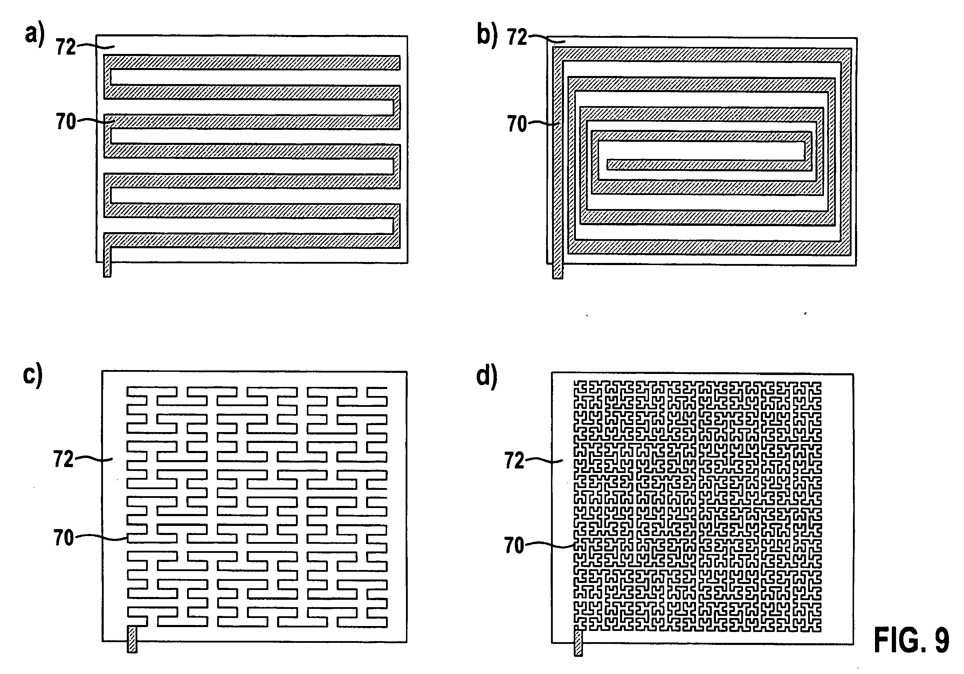

Figuren 9 a) bis d) zeigen verschiedene alternative Verläufe der jeweiligen Leiterbahn 70 auf dem flexiblen Trägermaterial 72. So zeigt Figur 9 a) einen Zickzackverlauf, während Figur 9 b) einen spiralförmigen Verlauf zeigt und die Figuren 9 c) und d verschiedene, mäanderförmige Verläufe der Leiterbahn 70.

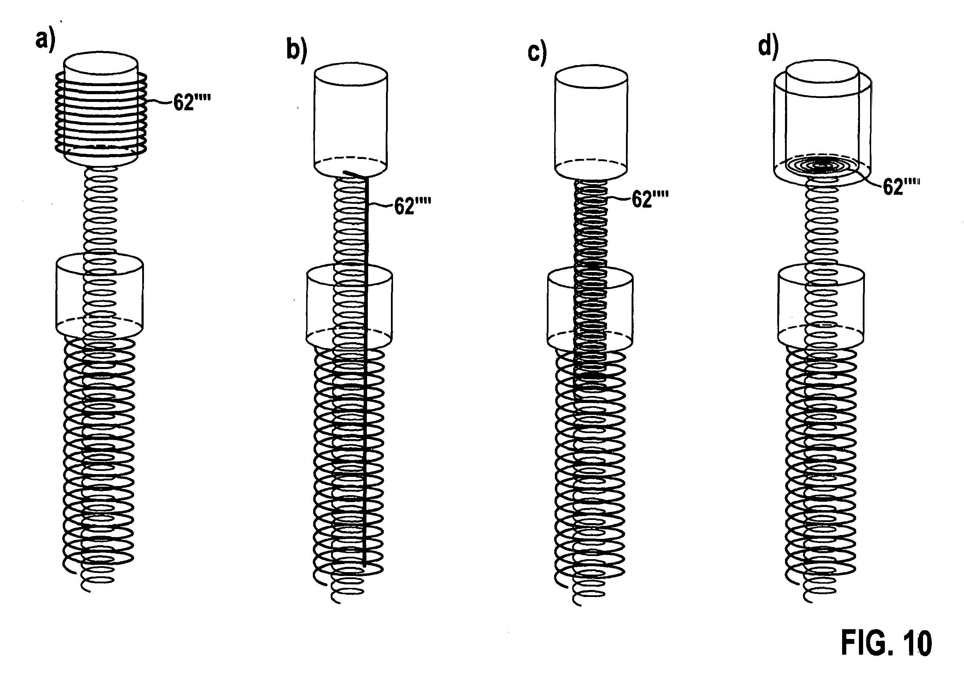

Figuren 10 a) bis 10 d) zeigen verschiedene Beispiele, wie der Leiter 62"" der zweiten elektrischen Komponente bei einer koaxialen Elektrodenleitung verlegt sein kann. Gemäß dem Ausführungsbeispiel in Figur 10 a) ist der Leiter 62"" helixförmig um einen distalen Elektrodenpol gewickelt. Gemäß dem Ausführungsbeispiel in Figur 10 b) verläuft der Leiter 62"" ausgehend vom distalen Elektrodenpol gestreckt in proximaler Richtung der Elektrodenleitung. Gemäß dem Ausführungsbeispiel in Figur 10 c) bildet der Leiter 62"" der zweiten elektrischen Komponente eine Helix, die koradial mit der Helix des Innenleiters, ausgehend vom distalen Elektrodenpol in proximale Richtung verläuft.

Gemäß dem Ausführungsbeispiel in Figur 10 d) ist der Leiter 62"" der zweiten elektrischen Komponente spiralförmig am proximalen Ende des distalen Elektrodenpols angeordnet.

Andere, nicht dargestellte Ausführungsvarianten schließen einen koradialen und einen mehrlumigen Aufbau der Elektrodenleitung ein. Bei einem mehrlumigen Aufbau werden die einzelnen Leiter nicht oder nur teilweise gewendelt. Der Leiter der zweiten elektrischen Komponente kann in diesem Fall ähnlich den Ausführungsbeispielen in Figuren 10 a) bis 10 d) geführt werden.

Weitere, in den Figuren z.T. nicht ausdrücklich dargestellte Ausführungsvarianten sind z.B. die Folgenden:

In weiteren Varianten ist der Draht als Leiter der zweiten elektrischen Komponente mäanderförmig, geflochten oder zufällig "geknäuelt" untergebracht.

In weiteren Varianten ist eine Litze bzw. ein Seil, das elektrisch leitend mit dem Ende des Elektrodenkopfes verbunden ist, als Leiter der zweiten elektrischen Komponente in die Kopfisolation eingebettet. Die Litze oder das Seil kann je nach weiterer Variante eine eigene Isolation besitzen oder nicht besitzen.

In weiteren Ausführungsformen geht der auf die Wellenlänge der eingekoppelten Hochfrequenzenergie abgestimmte Draht als Leiter der zweiten elektrischen Komponente in proximaler Richtung der Elektrodenleitung über den Elektrodenkopf hinaus und ist in die Isolation des anschließenden Elektrodenkörpers eingebettet.

In weiteren Ausführungsformen soll unter Elektrodenkopf der Verbindungspunkt einer Flächenelektrode z.B. zur Defibrillation des Herzens mit dem die Energie zuführenden Elektrodenleiter (dem Funktionsleiter) verstanden werden, wobei mit dem "Elektrodenkopf" ein isolierter Draht als Leiter der zweiten elektrischen Komponente je nach Ausführungsform mit eigener oder ohne eigene Isolation verbunden ist, der parallel zur Flächenelektrode gemäß den vorgenannten Prinzipien auf die Wellenlänge der eingekoppelten Hochfrequenzenergie abgestimmt ist.

In einer weiteren Ausführungsvariante besteht die zweite elektrische Komponente aus einem sehr dünnen Draht (50μm), der koaxial zur Zuleitung (d.h. der ersten elektrischen Komponente) um den Elektrodenkopf gewickelt wird, wobei die Wicklung eine sehr kleine Steigung <100μm aufweist.

Gemäß weiterer Ausführungsvarianten hat der weitergeführte elektrische Leiter folgende Leitungsgeometrie: gerade, gewickelt, doppelspiralförmig, spiralförmig, spiralförmig gewickelt, fraktal, mäanderförmig, geknäuelt oder einer Kombination der genannten Geometrien.

Geeignete Herstellverfahren zum Erzeugen der Leitungsgeometrie sind beispielsweise: Laserschneiden, Gießen, Wickeln, Bedrucken (z. B. Sieb- oder Tampondruck), Spanen (Fräsen, Drehen, Schleifen..), Ätzen, PVD, CVD, Sputtern, Prägen, galvanisches Abscheiden.

Geeignete Herstellverfahren für den dielektrisch wirksamen Körper bzw. die Kombination mit der Leitungsgeometrie sind beispielsweise:

Tauchbeschichtung, Sintern: der dielektrische Körper wird als Sinterkörper ausgelegt oder in Kombination mit dem elektrischen Leiter gesintert, Spritzgießen, Schmelzen, Extrusion, elektrostatisches Abscheiden (z.B. Pulverbeschichtung)

Konstruktiv umsetzbar sind n=0 bis n=2. Bei einem offenen (isolierten) Ende führt die Reflexion zu einem Knoten einer stehenden Welle am Punkt des Gewebekontaktes.

Vorteilhaft ist auch eine Zuleitung, die beispielsweise einen zentralen Leitungsabschnitt einer Elektrodenleitung bilden kann, bei deren erster elektrischer Komponente im Bereich zu erwartender Wellenbäuche jeweils eine zweite elektrische Komponente vorgesehen ist. Eine solche Zuleitung kann auf beliebige Länge für unterschiedliche Varianten zugeschnitten und nachträglich getrimmt werden.

Die zweite elektrische Komponente kann auch mehrere (zwei oder mehr) weiterführende Leiter verschiedener elektrischer Länge aufweisen, um somit bei mehreren eingekoppelten Frequenzen wirksam zu sein.

Der oder die Leiter der zweiten elektrischen Komponente kann/können entlang der Länge eine unterschiedliche Leiterbreite haben, um breitbandiger effektiv zu sein. Der oder die Leiter können beispielsweise sich (vom Kontaktpunkt ausgehend) linear oder in Stufen verjüngend ausgebildet sein.

Insbesondere bei Elektrophysiologie-Kathetern kann der Leiter der zweiten elektrischen Komponente mit Visualisierungsspulen (zur besseren Sichtbarkeit des distalen Endes des Katheters im MR-Bild) kombiniert sein.

Die Kombination aus weiterführendem elektrischen Leiter der zweiten elektrischen Komponente und Trägermaterial kann elektrisch mit dem Innenleiter verbunden und im distalen Bereich einer Elektrodenleitung

- außen auf dem Kopfgehäuse angebracht,

- innen in dem Kopfgehäuse angebracht,

- innerhalb des Kopfgehäuses auf einer Welle für eine korkenzieherartige Tip-Elektrode angebracht, oder

- unter einer Ringelektrode montiert

sein oder einen Abschnitt des Kopfgehäuses bilden

Als Trägermaterial für den Leiter der zweiten elektrischen Komponente (z.B. die Folie 72) und das Dielektrikum kommen beispielsweise Lack, Keramik, Glas, Polytetrafluoretylen oder dergleichen in Frage.

Dieser elektrische Leiter ist in einer besonderen Ausführung vom Punkt der Kontaktierung (Kontaktpunkt) des therapeutischen/diagnostischen Funktionsleiters in distale Richtung geführt, und zwar gerade, gewendelt oder mäanderartig.

Durch Verwendung dielektrisch wirksamer Einbettung der zweiten elektrischen Komponente sind miniaturisierte Umsetzungen möglich. Durch die Parallelschaltung hat die zweite elektrische Komponente keinen oder nur geringen Einfluss auf funktionelle Eigenschaften des Implantats. Mechanisch und elektrisch robuste Umsetzungen sind möglich und es sind nur geringe Änderungen bestehender Designkonzepte nötig. Die Wirksamkeit der zweiten elektrischen Komponente ist auch für verschiedene Frequenzen einer elektromagnetischen Welle gegeben. Gleichzeitig sind die Anforderungen an die Maßtoleranz gering.

**Patentansprüche**

1. Temporär oder permanent implantierbares medizinisches Gerät, mit mindestens einer langgestreckten elektrischen Leitung,
   die einen Funktionsleiter (42; 44) als erste elektrische Komponente oder als Teil einer ersten elektrischen Komponente enthält, wobei der Funktionsleiter (42, 44) mit wenigstens einem Elektrodenpol (22, 24) zur Abgabe von Therapiesignalen oder zur Erfassung von Diagnosesignalen verbunden ist; und
   die mindestens eine zweite elektrische Komponente (58, 62; 70, 72) enthält, die mit der ersten elektrischen Komponente elektrischen Kontakt hat und deren elektrische Länge mindestens einem Zehntel der Wellenlänge $\lambda$ von elektromagnetischen Wellen in einem Radiofrequenzbereich entspricht, wobei die zweite elektrische Komponente (58, 62; 70, 72) einen elektrischen Leiter (62, 70) aufweist, wobei ein Ende des Leiters (62, 70) elektrisch mit dem zumindest einen Elektrodenpol (22) verbunden ist,
   **dadurch gekennzeichnet, dass**
   ein zweites Ende des Leiters der zweiten elektrischen Komponente mit dem zumindest einen Elektrodenpol (22) kapazitiv, induktiv oder galvanisch verbunden ist.

2. Implantierbares medizinisches Gerät nach Anspruch 1, **dadurch gekennzeichnet, dass** das implantierbare medizinische Gerät eine Elektrodenleitung (20) zum Anschluss oder als Teil eines Therapie- und/oder Monitoringgerätes ist.

3. Implantierbares medizinisches Gerät nach Anspruch 2, **dadurch gekennzeichnet, dass** die Elektrodenleitung ein an ein Therapie- und/oder Monitoringgerät anzuschließendes oder angeschlossenes proximales Ende (80) und ein von diesem entferntes distales Ende (81) aufweist, wobei der zumindest eine Elektrodenpol (22) und die zweite elektrische Komponente (58, 62) am distalen Ende der Elektrodenleitung (20) angeordnet sind.

4. Implantierbares medizinisches Gerät nach Anspruch 2 oder 3, **dadurch gekennzeichnet, dass** die zweite elektrische Komponente (58, 62; 70, 72) um einen Längsabschnitt der Elektrodenleitung (20) gewickelt ist.

5. Implantierbares medizinisches Gerät nach wenigstens einem der Ansprüche 1 bis 4, **dadurch gekennzeichnet, dass** die zweite elektrische Komponente (58, 62; 70, 72) einen elektrischen Leiter (62; 70) aufweist, der mäanderartig gewunden oder helix- oder spiralartig gewickelt oder sowohl gewunden als auch gewickelt ist, so dass die zweite elektrische Komponente äußere Abmessungen aufweist, die wesentlich geringer sind, als die elektrische Länge des Leiters.

6. Implantierbares medizinisches Gerät nach wenigstens einem der Ansprüche 1 bis 5, **dadurch gekennzeichnet, dass** die erste elektrische Komponente (42; 44) ein proximales und ein distales Ende aufweist, und die erste und die zweite elektrische Komponente an einem Kontaktpunkt (56) miteinander elektrisch verbunden sind, der sich im Bereich des distalen Endes (81) der ersten elektrischen Komponente befindet.

7. Implantierbares medizinisches Gerät nach wenigstens einem der Ansprüche 1 bis 6, **dadurch gekennzeichnet, dass** die zweite elektrische Komponente (58, 62; 70, 72) und/oder deren elektrischer Leiter (62; 70) ganz oder teilweise von einem dielektrisch wirksamen Medium umgeben ist, das eine relative Permittivität $\varepsilon_R$ größer 10 hat.

8. Implantierbares medizinisches Gerät nach Anspruch 7, **dadurch gekennzeichnet, dass** das dielektrisch wirksame Medium ganz oder abschnittsweise aus einem Ferroelektrikum und/oder einem der folgenden Werkstoffe oder einer Kombination davon besteht: Glas, Bariumtitanat, Polyethylen, Siliziumoxid, Siliziumkarbid, Keramik, Piezowerkstoffe, Aluminiumoxid, Titanoxid, Tantaloxid, Halbleiterwerkstoffe, Kunststoffe mit Beimengungen der oben genannten Werkstoffe.

9. Implantierbares medizinisches Gerät nach wenigstens einem der Ansprüche 1 bis 8, **dadurch gekennzeichnet, dass** die zweite elektrische Komponente (58, 62; 70, 72) für einen Gewebekontakt im Abstand $n * \lambda/2$ einer Wellenlänge $\lambda$ im Radiofrequenzbereich von mindestens einem Leitungsende der zweiten elektrischen Komponente ausgebildet ist, wobei n eine natürliche Zahl im Bereich von n=1 bis n=2 ist.

10. Implantierbares medizinisches Gerät nach Anspruch 9 und 7 oder 8, **dadurch gekennzeichnet, dass** die Kombination aus zweiter elektrischer Komponente und dielektrischem Medium zusätzlich in Kontakt mit einer elektrisch leitfähigen Fläche steht.

11. Implantierbares medizinisches Gerät nach wenigstens einem der Ansprüche 1 bis 10, **dadurch gekennzeichnet, dass** die elektrische Länge der zweiten elektrischen Komponente (58, 62; 70, 72) auf einen Frequenzbereich zwischen 10 MHz und 400MHz und vorzugsweise zwischen 40 MHz und 150 MHz abgestimmt ist.

12. Implantierbares medizinisches Gerät nach wenigstens einem der Ansprüche 1 bis 11, **dadurch gekennzeichnet, dass** die zweite elektrische Komponente (58, 62; 70, 72) einen Leiter (62; 70) aufweist, der entlang seiner Länge oder eines Teils davon isoliert ist.

13. Implantierbares medizinisches Gerät nach wenigstens einem der Ansprüche 1 bis 12, **dadurch gekennzeichnet, dass** die zweite elektrische Komponente (58, 62; 70, 72) eine elektrische Länge hat, die einem Viertel der Wellenlänge ($\lambda$/4) elektromagnetischer Wellen im Frequenzbereich von 40MHz bis 150MHz entspricht

14. Implantierbares medizinisches Gerät nach wenigstens einem der Ansprüche 1 bis 13, **dadurch gekennzeichnet, dass** die zweite elektrische Komponente (70, 72) eine Leiterbahn 70 als Leiter aufweist, die auf ein flexibles Trägermaterial (72) aufgebracht ist, welches um die erste elektrische Komponente gewickelt ist.

**Claims**

1. A temporarily or permanently implantable medical device, comprising at least one elongate electrical line,

   - which contains a functional conductor (42; 44) as a first electrical component or as part of a first electrical component, wherein the functional conductor (42, 44) is connected to at least one electrode pole (22, 24) for delivery of therapy signals or for detection of diagnosis signals; and
   - which contains at least one second electrical component (58, 62; 70, 72), which has electrical contact with the first electrical component and of which the electrical length corresponds to at least a tenth of the wavelength $\lambda$ of electromagnetic waves in a radiofrequency range, wherein the second electrical component (58, 62; 70, 72) has an electrical conductor (62; 70), wherein one end of the conductor (62, 70) is electrically connected to the at least one electrode pole (22),

   **characterised in that**
   a second end of the conductor of the second electrical component is capacitively, inductively or galvanically connected to the at least one electrode pole (22).

2. The implantable medical device according to Claim 1, **characterised in that** the implantable medical device is an electrode line (20) for connection to a therapy and/or monitoring device or as part of a therapy and/or monitoring device.

3. The implantable medical device according to Claim 2, **characterised in that** the electrode line has a proximal end (80) that is to be connected or that is connected to a therapy and/or monitoring device and a distal end (81) remote therefrom, wherein the at least one electrode pole (22) and the second electrical component (58, 62) are arranged at the distal end of the electrode line (20).

4. The implantable medical device according to Claim 2 or 3, **characterised in that** the second electrical component (58, 62; 70, 72) is wound around a longitudinal portion of the electrode line (20).

5. The implantable medical device according to at least one of Claims 1 to 4, **characterised in that** the second electrical component (58, 62; 70, 72) has an electrical conductor (62; 70) which is coiled in a meandering manner or is helically or spirally wound or is both coiled and wound, such that the second electrical component has outer dimensions that are substantially smaller than the electrical length of the conductor.

6. The implantable medical device according to at least one of Claims 1 to 5, **characterised in that** the first electrical component (42; 44) has a proximal and a distal end, and the first and the second electrical component are electrically interconnected at a contact point (56) located in the region of the distal end (81) of the first electrical component.

7. The implantable medical device according to at least one of Claims 1 to 6, **characterised in that** the second electrical component (58, 62; 70, 72) and/or the electrical conductor thereof (62; 70) is surrounded completely or in part by a dielectrically effective medium which has a relative permittivity $\varepsilon_R$ greater than 10.

8. The implantable medical device according to Claim 7, **characterised in that** the dielectrically effective medium consists completely or in portions of a ferroelectric and/or one of the following materials or a combination thereof: glass, barium titanate, polyethylene, silicon oxide, silicon carbide, ceramic, piezo materials, aluminium oxide, titanium oxide, tantalum oxide, semiconductor materials, and plastics with additions of the above-mentioned materials.

9. The implantable medical device according to at least one of Claims 1 to 8, **characterised in that** the second electrical component (58, 62; 70, 72) is designed for tissue contact at a distance $n * \lambda/2$ of a wavelength $\lambda$ in the radiofrequency range from at least one line end of the second electrical component, wherein n is a natural number in the range from n=1 to n=2.

10. The implantable medical device according to Claim 9 and 7 or 8, **characterised in that** the combination of second electrical component and dielectric medium is additionally in contact with an electrically conductive surface.

11. The implantable medical device according to at least one of Claims 1 to 10, **characterised in that** the electrical length of the second electrical component (58, 62; 70, 72) is matched to a frequency range between 10 MHz and 400 MHz and preferably between 40 MHz and 150 MHz.

12. The implantable medical device according to at least one of Claims 1 to 11, **characterised in that** the second electrical component (58, 62; 70, 72) has a conductor (62; 70), which is insulated along its length or part thereof.

13. The implantable medical device according to at least one of Claims 1 to 12, **characterised in that** the second electrical component (58, 62; 70, 72) has an electrical length that corresponds to a quarter of the wavelength ($\lambda/4$) of electromagnetic waves in the frequency range from 40 MHz to 150 MHz.

14. The implantable medical device according to at least one of Claims 1 to 13, **characterised in that** the second electrical component (70, 72) has a conductive track (70) as conductor, which is applied to a flexible support material (72) which is wound around the first electrical component.

## Revendications

1. Appareil médical implantable de manière temporaire ou permanente, avec au moins une ligne électrique tirée en longueur,
   qui contient un conducteur fonctionnel (42 ; 44) sous la forme d'une première composante électrique ou sous la forme d'une partie d'une première composante électrique, où le conducteur fonctionnel (42, 44) est relié avec au moins un pôle d'électrode (22, 24) pour la délivrance de signaux thérapeutiques ou pour la détection de signaux de diagnostic; et
   qui contient au moins une deuxième composante électrique (58, 62 ; 70, 72) qui a un contact électrique avec la première composante électrique et dont la longueur électrique correspond à au moins un dixième de la longueur d'onde $\lambda$ d'ondes électromagnétiques dans un domaine de fréquences radio, où la deuxième composante électrique (58, 62 ; 70, 72) présente un conducteur électrique (62, 70) où une extrémité du conducteur (62, 70) est reliée électriquement avec l'au moins un pôle d'électrode (22),
   **caractérisé en ce**
   **qu'**une deuxième extrémité du conducteur de la deuxième composante électrique est reliée de manière capacitive, inductive ou galvanique avec l'au moins un pôle d'électrode (22).

2. Appareil médical implantable selon la revendication 1, **caractérisé en ce que** l'appareil médical implantable est une ligne d'électrode (20) pour le raccordement, ou est sous forme d'une partie, d'un appareil de thérapie et/ou de surveillance.

3. Appareil médical implantable selon la revendication 2, **caractérisé en ce que** la ligne d'électrode présente une extrémité proximale (80) faisant suite, ou raccordée, à un appareil de thérapie et/ou de surveillance et une extrémité distale (81) éloignée de celle-ci, où l'au moins un pôle électrique (22) et la deuxième composante électrique (58, 62) sont disposées à l'extrémité distale de la ligne d'électrode (20).

**4.** Appareil médical implantable selon les revendications 2 ou 3, **caractérisé en ce que** la deuxième composante électrique (58, 62 ; 70, 72) est enroulée autour d'une section longitudinale de la ligne d'électrode (20).

**5.** Appareil médical implantable selon au moins une des revendications 1 à 4, **caractérisé en ce que** la deuxième composante électrique (58, 62 ; 70, 72) présente un conducteur électrique (62 ; 70) qui est enroulé sous forme de méandres, ou d'hélice, ou de spirale, ou peut être aussi bien entortillé qu'enroulé, de sorte que la deuxième composante électrique présente des dimensions extérieures qui sont substantiellement inférieures à la longueur électrique du conducteur.

**6.** Appareil médical implantable selon au moins une des revendications 1 à 5, **caractérisé en ce que** la première composante électrique (42 ; 44) présente une extrémité proximale et une extrémité distale, et les première et deuxième composantes électriques sont reliées électriquement ensemble à un point de contact (56) qui se situe dans la zone de l'extrémité distale (81) de la première composante électrique.

**7.** Appareil médical implantable selon au moins une des revendications 1 à 6, **caractérisé en ce que** la deuxième composante électrique (58, 62 ; 70, 72) et/ou leurs conducteurs électriques (62 ; 70) sont totalement ou partiellement entourés d'un milieu efficace diélectrique qui a une permittivité relative $\varepsilon_R$ supérieure à 10.

**8.** Appareil médical implantable selon la revendication 7, **caractérisé en ce que** le milieu efficace diélectrique est constitué totalement ou sur des portions d'un matériau ferroélectrique et/ou d'une des matières suivantes ou d'une combinaison de celles-ci : verre, titanate de baryum, polyéthylène, oxyde de silicium, carbure de silicium, céramique, matières piézoélectriques, oxyde d'aluminium, oxyde de titane, oxyde de tantale, matières semi-conductrices, matières synthétiques avec des ajouts des produits cités ci-dessus.

**9.** Appareil médical implantable selon au moins une des revendications 1 à 8, **caractérisé en ce que** la deuxième composante électrique (58, 62 ; 70, 72) est conçue pour un contact avec des tissus à la distance n*λ/2 d'une longueur d'onde λ dans le domaine de fréquences radio d'au moins une extrémité de ligne de la deuxième composante électrique, où n est un nombre naturel dans la gamme n = 1 à n = 2.

**10.** Appareil médical implantable selon les revendications 9 et 7 ou 8, **caractérisé en ce que** la combinaison de la deuxième composante électrique et du milieu diélectrique est en contact de manière complémentaire avec une surface électriquement conductrice.

**11.** Appareil médical implantable selon au moins une des revendications 1 à 10, **caractérisé en ce que** la longueur électrique de la deuxième composante électrique (58, 62 ; 70, 72) est adaptée à un domaine de fréquences entre 10 MHz et 400 MHz, et de préférence, entre 40 MHz et 150 MHz.

**12.** Appareil médical implantable selon au moins une des revendications 1 à 11, **caractérisé en ce que** la deuxième composante électrique (58, 62 ; 70, 72) présente un conducteur (62 ; 70) qui est isolé le long de sa longueur ou d'une partie de celle-ci.

**13.** Appareil médical implantable selon au moins une des revendications 1 à 12, **caractérisé en ce que** la deuxième composante électrique (58, 62 ; 70, 72) a une longueur électrique qui correspond à un quart de la longueur d'onde (λ/4) des ondes électromagnétiques dans le domaine de fréquences de 40 MHz à 150 MHz.

**14.** Appareil médical implantable selon au moins une des revendications 1 à 13, **caractérisé en ce que** la deuxième composante électrique (70, 72) présente une bande conductrice (70) servant de conducteur, qui est rapportée sur un matériau support (72) souple, lequel est enroulé autour de la première composante électrique.

FIG. 1

FIG. 2

EP 2 848 282 B1

FIG. 3

EP 2 848 282 B1

**FIG. 4**

22"

60

58"

56"

62"

81

EP 2 848 282 B1

FIG. 5

17

EP 2 848 282 B1

FIG. 6

FIG. 7

18

a)

72    70

30 μm

30 μm

30 μm

30 μm

70 μm

b)

3 mm

50 mm

c)

5 μm

3 mm

d)

FIG. 8

EP 2 848 282 B1

FIG. 9

FIG. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130204335 A1
- EP 2486952 A1
- US 20120078333 A1
- US 20120109261 A1
- US 20100211129 A1
- US 20080243218 A1
- US 20080033497 A1